# EUROPEAN PATENT SPECIFICATION

(11) **EP 4 259 597 B1**
(45) Date of publication and mention of the grant of the patent: **24.07.2024**
(21) Application number: 21802367.9
(22) Date of filing: 05.11.2021
(51) Int. Cl.: C07C 29/147, C11D 1/12, C11D 1/14, C11D 1/29, C11D 3/20, C07C 67/03

(54) **PROCESS TO PRODUCE OLEYL ALCOHOL**
VERFAHREN ZUR HERSTELLUNG VON OLEYLALKOHOL
PROCÉDÉ DE PRODUCTION D'ALCOOL D'OLÉYLE

(30) Priority: 08.12.2020 EP 20212402
(43) Date of publication of application: 18.10.2023
(73) Proprietor: Unilever IP Holdings B.V., 3013 AL Rotterdam (NL); Unilever Global IP Limited, Wirral, Merseyside CH62 4ZD (GB)
(72) Inventor: BATCHELOR, Stephen, Norman, Wirral Merseyside CH63 3JW (GB)
(74) Representative: McHugh, Paul Edward
(86) International application number: PCT/EP2021/080769
(87) International publication number: WO 2022/122267

(56) References cited:
- CA-A1- 2 762 583
- US-A- 5 672 781
- US-A1- 2013 281 688
- US-B1- 6 229 056
- WORLD HEALTH ORG. ET AL: "STANDARD FOR NAMED VEGETABLE OILS: Codex Alimentarius", 31 December 2019 (2019-12-31), XP055806497, Retrieved from the Internet <URL:http://www.fao.org/fao-who-codexalimentarius/sh-proxy/en/?lnk=1&url=https%3A%2F%2Fworkspace.fao.org%2Fsites%2Fcodex%2FStandards%2FCXS+210-1999%2FCXS_210e.pdf> [retrieved on 20210521]
- BRYAN R. MOSER: "Influence of Blending Canola, Palm, Soybean, and Sunflower Oil Methyl Esters on Fuel Properties of Biodiesel +", ENERGY & FUELS, vol. 22, no. 6, 19 November 2008 (2008-11-19), pages 4301 - 4306, XP055069553, ISSN: 0887-0624, DOI: 10.1021/ef800588x

## Description

### Field of Invention

The present invention concerns a process to produce oleyl alcohol, a useful intermediate in the production of surfactants.

### Background of the Invention

Oleyl based surfactants such as oleyl ether carboxylates, oleyl ether sulfates, oleyl alcohol ethoxylates and oleyl sulfates may be used in laundry detergents. The surfactants may be synthesised from oleyl alcohol, which is obtainable from palm oil or palm kernel oil by converting the triglyceride to the fatty acid or methyl esters then distilling to give the oleic fraction, followed by reduction (hydrogenation) of the carboxylic acid or methyl ester to the alcohol. The oleyl alcohol produced is a mixture of predominately oleyl alcohol with significant levels of palmyl alcohol and stearyl alcohol. The balance of these ingredient is important in controlling the cleaning and physical form of the surfactant.

US 5,672,781 (Henkel) discloses a fatty alcohol produced from palm kernel oil where the palm kernel oil is transesterfied to the methyl ester followed by fractionation by distillation and then hydrogenation to give the fatty alcohol. Also disclosed is fatty alcohol produced from a 70:30 wt.% mixture of palm kernel oil methyl ester and rapeseed oil methyl esters produced by a similar process. The processes require fractionation by distillation to high temperatures, which requires considerable energy for heating. The process also produces waste fractions.

US 6,229,056 discloses a process to obtain fatty alcohol from high oleic sunflower oils. This process includes a last distillation step. US2013/281688 discloses dishwashing compositions wherein oleyl alcohol ethoxylate may be present.

There is need for an improved process to produce oleyl alcohol and the surfactants synthesised from it, there is a need to produce such materials using simpler chemical processes, containing fewer highly energetic steps and producing fewer waste fractions. Surprisingly, this problem can be solved by the process and oleyl alcohol of the invention.

### Summary of the Invention

According to the invention, there is provided a process to produce an oleyl alcohol comprising the following steps:-
a) hydrogenation of a fatty acid, a fatty acid methyl ester or a combination thereof to provide a fatty alcohol;
wherein the fatty acid, the fatty acid methyl ester oil or combination thereof contain oleyl chains obtained from a mixture of 1:9 to 6:4, preferably 2:8 to 5:5 oil (1) and oil (2), where oil (1) is selected from palm oil, cottonseed oil or mixtures thereof, oil (2) is selected from tall oil, olive oil, low erucic rapeseed oil, sunflower seed oil variants containing greater than 60 wt.% oleic acid, or mixtures thereof, wherein the process does not include distillation.

Preferably in the process, oil (1) is palm oil, and oil (2) is selected from low erucic rapeseed oil and sunflower seed oil variants containing greater than 60 wt.% oleic acid, or mixtures thereof, preferably wherein oil (2) is low erucic rapeseed oil.

Preferably in the process, the oleyl alcohol is converted to a surfactant, preferably to a surfactant selected from: oleyl sulfate, oleyl alcohol ethoxylate, oleyl ether sulfate, oleyl ether carboxylate, and oleyl sulfosuccinate, preferably oleyl alcohol ethoxylate, oleyl ether sulfate and oleyl ether carboxylate.

Preferably in the process, the fatty acid methyl ester is produced by transesterification of triglyceride to fatty acid methyl esters followed by removal of glycerol.

### Detailed Description of the Invention

The indefinite article "a" or "an" and its corresponding definite article "the" as used herein means at least one, or one or more, unless specified otherwise.

wt.% relates to the amount by weight of the ingredient based on the total weight of the composition. For anionic surfactants, wt.% is calculated based on the protonated form of the surfactant.

### Production of the oleyl alcohol

Herein is provided a process to produce an oleyl alcohol comprising the following steps:-
a) hydrogenation of a fatty acid, a fatty acid methyl ester or a combination thereof to provide a fatty alcohol;
wherein the fatty acid, the fatty acid methyl ester oil or combination thereof contain oleyl chains obtained from a mixture of 1:9 to 6:4, preferably 2:8 to 5:5 oil (1) and oil (2), where oil (1) is selected from palm oil, cottonseed oil or mixtures thereof, oil (2) is selected from tall oil, olive oil, low erucic rapeseed oil, sunflower seed oil variants containing greater than 60 wt.% oleic acid, or mixtures thereof.

### Surfactant production from the oleyl alcohol

The oleyl alcohol is a useful intermediate in the production of various surfactants. Examples of oleyl based surfactants follow.

### Hydrogenation

Fatty alcohols are usually made by reducing the corresponding fatty acids or esters, typically by catalytic hydrogenation. Fatty Alcohol may be produced from fatty acid methyl esters by hydrogenated in the presence of chromium- or zinc-containing mixed catalysts typically at 250-350°C and hydrogen pressures of from 200 to 275 bar.

For example, using a CuCrO₄ catalyst, fatty acid methyl esters may be hydrogenated to the alcohol in a typical hydrogenation reactor under a pressure of around 225 bar and at a temperature of 275 to 330°C. The use of copper chromite is described by Homer Adkins in Catalytic Hydrogenation of Esters to Alcohols (2011 Organic Reactions, Inc. Published by John Wiley and Sons, Inc). Processes are also discussed in Sanchez M.A. et al J.Chem. Technol. Biotechnol 2017; 92:27-92; Thakur, D.S. et al JAOCS vol 76 (1999) 995-1000 and Ullmann's Enzyclopaedie der technischen Chemie, Verlag Chemie, Weinheim, 4th Edition, Vol. 11, pages 436 et seq. Other catalysts based on transition metal such as Ru or Fe may be used as described in Organometallics 2015, 34, 1, 335-339; Chimica Oggi Catalysis & Biocatalysis 2019, 37(4), 10. Lindlar catalyst (palladium on calcium carbonate; poisoned with lead) may be used ACS Omega 2020, 5, 36, 22901-22913.

Preferably the cis configuration of the double bond on the oleyl chain is maintained.

Preferably the alcohol of the invention has a C16 fraction of 10 to 28 wt.% and a C18 fraction of greater than 70 wt.%. Preferably the C18 fraction is greater than 50 wt.%, more preferably greater than 70 wt.% mono-unsaturated.

The conjugene content is preferably less than 6 wt.%, more preferably less than 4 wt.% most preferably less than 2 wt.%. Preferably the levels of linoleyl alcohol is less than 6 wt.%, more preferably less than 4 wt.%, most preferably less than 2 wt.%. Preferably the level of linolenyl alcohol is less than 4 wt.%, more preferably less than 1 wt.%, most preferably less than 0.4 wt.%.

### Formation of Fatty Acid

Fatty acids may be formed from triglyceride by alkaline or enzymatic hydrolysis, followed by removal of the glycerol.

### Transesterification

Transesterification reactions of a triglyceride to fatty acid methyl esters and glycerol are discussed in Fattah et al (Front. Energy Res., June 2020, volume 8 article 101) and references therein. In the transesterification process a glyceride reacts with methanol in the presence of a catalyst forming fatty acid methyl esters and glycerol. A strong base or a strong acid can be used as a catalyst, for NaOH, KOH, sodium or potassium methoxide.

In a typical process alcohol, alkaline catalyst and oil are combined in a reactor and agitated for approximately an hour at 60°C. A batch reactor may be used or continuous flow processes involving continuous stirred-tank reactors (CSTR) or plug flow reactors. Following the reaction, the glycerol is removed from the methyl esters. Due to the low solubility of glycerol in the esters, this separation generally occurs quickly and may be accomplished with either a settling tank or a centrifuge.

After separation from the glycerol, the methyl esters pass through a methanol stripper, usually a vacuum flash process or a falling film evaporator, before entering a neutralization step and water washing. Acid is added to neutralize any residual catalyst and to split any soap that may have formed during the reaction. Soaps will react with the acid to form water-soluble salts and free fatty acids. The salts will be removed during the water washing step and the free fatty acids will stay in the methyl ester.

### Triglyceride source

Palm oil for use in the invention is selected from Crude Palm oil and the refined bleached deoderized Palm Oil or neutralized bleached deodorized Palm Oil derived there from, including the fractionated derivatives Stearin and Olein.

Crude Palm oil is the oil extracted from the pulp (mesocarp) of fruit of oil palms, preferably the African oil palm *Elaeis guineensis.* The Palm oil is preferably non-deforestaion, non-peat and non-explotation Palm oil.

Palm oil is discussed in Palm Oil (1st Edition) Production, Processing, Characterization, and Uses, edited by Oi-Ming Lai Chin-Ping Tan Casimir Akoh(Academic Press and AOCS Press 2012).

Low erucic rapeseed oil (canola) are discussed in Canola and Rapeseed edited by F. Shahidi (Springer 1990). The high oleic acid variant may be used. The oil may be partially hydrogenated to reduce the level of polyunsaturated fatty acids it contains, however the oleic acid fraction should not be substantially reduced. Low erucic rapeseed oil contains a maximum of 2 wt.%, preferably 0.5 wt.% of erucic acid.

Standard sunflower oil is naturally rich in polyunsaturated linoleic acid that makes up about 70% of the total sunflower oil content, and the second most abundant is monounsaturated oleic acid contributing with 20%. Mid oleic, high oleic, high stearic/high oleic sunflower oils are variants where the oil contain greater than 60 wt.% oleic acid. The high oleic and high stearic/high oleic variant are more preferred, and high oleic most preferred. The fatty acid composition of high oleic sunflower oil is typically 82% oleic, 9% linoleic and 9% saturates. The fatty acid composition of high stearic/high oleic sunflower oil is typically 72% oleic, 5% linoleic, 18% stearic and 5% other saturates.

Cottonseed oil is the seeds of cotton plants of various species, mainly Gossypium hirsutum and Gossypium herbaceum.

Olive oil is obtained from olives, the fruit of Olea europaea; family Oleaceae. Lampante oil, non-edible olive oil is a preferred type.

The oleyl alcohol of the invention, wherein the oleyl chain is obtained from a mixture of 1:9 to 6:4, preferably 2:8 to 5:5 of oil (1) : oil (2) by hydrogenation of a fatty acid, a fatty acid methyl ester or a combination thereof to provide a fatty alcohol, where oil (1) is selected from palm oil, cottonseed oil or a mixture thereof, and oil (2) is selected from tall oil, olive oil, low erucic rapeseed oil, sunflower seed oil variants containing greater than 60 wt.% oleic acid, or mixtures thereof.

Preferably oil (1) is palm oil, and oil (2) is selected from low erucic rapeseed oil and sunflower seed oil variants containing greater than 60 wt.% oleic acid, or mixtures thereof, more preferably low erucic rapeseed oil.

Preferred iodine values are from 65 to 85, more preferably from 65 to 75, most preferably from 70 to 75.

### Oleyl based surfactant

The oleyl alcohol is converted to surfactants by chemically linking a water-soluble head group to the OH group, preferably selected from sulfate, ethoxylate, ether sulfate, ether carboxylate, sulfosuccinate, more preferably ethoxylate, ether sulfate, ether carboxylate.

Anionic surfactants are discussed in Anionic Surfactants Organic Chemistry edited by Helmut W. Stache (Marcel Dekker 1996)

The integers 'q' 'm' and 'n' are mole average values.

### Oleyl alcohol ethoxylate

Oleyl alcohol ethoxylates may be synthesised by ethoxylation of the oleyl alcohol, R₁-OH, via the reaction:

R₁-OH + *q* ethylene oxide → R₁-O-(CH₂CH₂O)_{q}-H

The oleyl alcohol ethoxylate preferably has q is from 4 to 20, more preferably 5 to 14, most preferably 8, 9, 10, 11, 12.

Ethoxylation reactions are described in Non-lonic Surfactant Organic Chemistry (N. M. van Os ed), Surfactant Science Series Volume 72, CRC Press.

Preferably the ethoxylation reactions are base catalysed using NaOH, KOH, or NaOCH₃. Even more preferred are catalyst which provide narrower ethoxy distribution than NaOH, KOH, or NaOCH₃. Preferably these narrower distribution catalysts involve a Group II base such as Ba dodecanoate; Group II metal alkoxides; Group II hyrodrotalcite as described in WO 2007/147866. Lanthanides may also be used.

Preferably the narrow ethoxy distribution has greater than 70 wt.%, more preferably greater than 80 wt.% of the alcohol ethoxylate R-O-(CH₂CH₂O)_{q}-H in the range R-O-(CH₂CH₂O)ₓ-H to R-O-(CH₂CH₂O)_{y}-H where q is the mole average degree of ethoxylation and x and y are absolute numbers, where x = q - (q/2) and y = q + (q/2). For example, when q = 10, then greater than 70 wt.% of the alcohol ethoxylate should consist of ethoxylate with 5, 6, 7, 8, 9 10, 11, 12, 13, 14 and 15 ethoxylate groups.

### Oleyl ether sulfate

The oleyl ether sulfate is preferably of the form:

Ri-O-(CH₂CH₂O)ₘ-SO₃H

Where m is preferably from 3 to 20, preferably 4 to 12, more preferably 5, 6, 7, 8.

Ether sulfates are discussed in the Anionic Surfactants: Organic Chemistry edited by Helmut W. Stache (Marcel Dekker 1996), Surfactant Science Series published by CRC press. Ether sulfate may be synthesised by the sulphonation of the corresponding alcohol ethoxylate.

### Oleyl ether carboxylate

The oleyl ether carboxylate is preferably of the form

R₁-O-(CH₂CH₂O)ₙ-CH₂COOH

Preferably n is from 5 to 20, more preferably from 6 to 14, most preferably from 8, 9, 10, 11, 12.

Alkyl ether carboxylic acids synthesis is discussed in Anionic Surfactants Organic Chemistry edited by Helmut W. Stache (Marcel Dekker 1996). They may be synthesised via the reaction of the corresponding alcohol ethoxylate with chloroacetic acid or monochloro sodium acetate in the presence of NaOH.

### Liquid laundry detergents

In one embodiment, the product obtained by the process of the invention takes the form of a detergent composition, preferably a liquid laundry detergent composition. Such compositions are explained in greater detail in the following paragraphs.

The term "laundry detergent" in the context of this invention denotes formulated compositions intended for and capable of wetting and cleaning domestic laundry such as clothing, linens and other household textiles. The aim is to provide a composition which on dilution is capable of forming a liquid laundry detergent composition and in the manner now described.

The term "linen" is often used to describe certain types of laundry items including bed sheets, pillow cases, towels, tablecloths, table napkins and uniforms. Textiles can include woven fabrics, non-woven fabrics, and knitted fabrics; and can include natural or synthetic fibres such as silk fibres, linen fibres, cotton fibres, polyester fibres, polyamide fibres such as nylon, acrylic fibres, acetate fibres, and blends thereof including cotton and polyester blends.

Examples of liquid laundry detergents include heavy-duty liquid laundry detergents for use in the wash cycle of automatic washing machines, as well as liquid fine wash and liquid colour care detergents such as those suitable for washing delicate garments (e.g. those made of silk or wool) either by hand or in the wash cycle of automatic washing machines.

The term "liquid" in the context of this invention denotes that a continuous phase or predominant part of the composition is liquid and that the composition is flowable at 15°C and above. Accordingly, the term "liquid" may encompass emulsions, suspensions, but not compositions having flowable yet stiffer consistency, known as gels or pastes. The viscosity of the composition is preferably from 100 to about 1,000 mPa.s at 25°C at a shear rate of 21 sec⁻¹. This shear rate is the shear rate that is usually exerted on the liquid when poured from a bottle. Pourable liquid detergent compositions preferably have a viscosity of from 200 to 500 mPa.s, preferably from 200 to 300 mPa.s.

Preferably, the liquid laundry detergent composition comprises from 50% water, more preferably from 70% wt. water and most preferably from 75% water. Preferably the water used has a french hardness of less than 5 degrees french hard, most preferably it is demineralised. Preferably the water is treated with a disinfectant, preferably selected from a chlorine based disinfectant, ozone or UV treatment, to sterilize the water.

A liquid laundry detergent composition may suitably have an aqueous continuous phase. By "aqueous continuous phase" is meant a continuous phase which has water as its basis. Preferably the formulation has a pH of 5 to 10, more preferably 6 to 8, most preferably 6.1 to 7.0

A liquid laundry detergent composition suitably comprises from 5 to 60% and preferably from 10 to 40% (by weight based on the total weight of the composition) of one or more detersive surfactants. Preferably greater than 50 wt.%, more preferably greater than 80 wt.%, most preferably great than 95 wt.% of the detersive surfactant are selected from oleyl based surfactants, more preferably oleyl ethoxylate, oleyl ether sulfate, oleyl ether carboxylate, most preferably oleyl ethoxylate and oleyl ether sulfate.

The term "detersive surfactant" in the context of this invention denotes a surfactant which provides a detersive (i.e. cleaning) effect to laundry treated as part of a domestic laundering process.

Preferably, the selection and amount of surfactant is such that the composition and the diluted mixture are isotropic in nature.

Preferably, the weight ratio of non-ionic surfactant to alkyl ether sulphate surfactant (wt. non-ionic / wt. alkyl ether sulphate) is from 0.5 to 2, preferably from 0.7 to 1.5, most preferably 0.9 to 1.1.

Preferably, the weight ratio of non-ionic surfactant to linear alkyl benzene sulphonate, where present, (wt. non-ionic/ wt. linear alkyl benzene sulphonate) is from 0.1 to 2, preferably 0.3 to 1, most preferably 0.45 to 0.85.

### ANTI-FOAM

The composition may also comprise an anti-foam. Anti-foam materials are well known in the art and include silicones and fatty acid.

Preferably, fatty acid soap is present at from 0 to 3.0% wt. of the composition, more preferably from 0 to 0.5% wt. and most preferably zero.

Suitable fatty acids in the context of this invention include aliphatic carboxylic acids of formula RCOOH, where R is a linear or branched alkyl or alkenyl chain containing from 6 to 24, more preferably 10 to 22, most preferably from 12 to 18 carbon atoms and 0 or 1 double bond. Preferred examples of such materials include saturated C12-18 fatty acids such as lauric acid, myristic acid, palmitic acid or stearic acid; and fatty acid mixtures in which 50 to 100% (by weight based on the total weight of the mixture) consists of saturated C12-18 fatty acids. Such mixtures may typically be derived from natural fats and/or optionally hydrogenated natural oils (such as coconut oil, palm kernel oil or tallow).

The fatty acids may be present in the form of their sodium, potassium or ammonium salts and/or in the form of soluble salts of organic bases, such as mono-, di- or triethanolamine.

Mixtures of any of the above described materials may also be used.

For formula accounting purposes, in the formulation, fatty acids and/or their salts (as defined above) are not included in the level of surfactant or in the level of builder.

### POLYMERIC CLEANING BOOSTERS

Anti-redeposition polymers stabilise the soil in the wash solution thus preventing redeposition of the soil. Suitable soil release polymers for use in the invention include alkoxylated polyethyleneimines. Polyethyleneimines are materials composed of ethylene imine units -CH₂CH₂NH- and, where branched, the hydrogen on the nitrogen is replaced by another chain of ethylene imine units. Preferred alkoxylated polyethyleneimines for use in the invention have a polyethyleneimine backbone of about 300 to about 10000 weight average molecular weight (M_{w}). The polyethyleneimine backbone may be linear or branched. It may be branched to the extent that it is a dendrimer. The alkoxylation may typically be ethoxylation or propoxylation, or a mixture of both. Where a nitrogen atom is alkoxylated, a preferred average degree of alkoxylation is from 10 to 30, preferably from 15 to 25 alkoxy groups per modification. A preferred material is ethoxylated polyethyleneimine, with an average degree of ethoxylation being from 10 to 30, preferably from 15 to 25 ethoxy groups per ethoxylated nitrogen atom in the polyethyleneimine backbone.

Mixtures of any of the above described materials may also be used.

A liquid laundry detergent composition will preferably comprise from 0.025 to 8% wt. of one or more anti-redeposition polymers such as, for example, the alkoxylated polyethyleneimines which are described above.

### SOIL RELEASE POLYMERS

Soil release polymers help to improve the detachment of soils from fabric by modifying the fabric surface during washing. The adsorption of a SRP over the fabric surface is promoted by an affinity between the chemical structure of the SRP and the target fibre.

SRPs for use in the invention may include a variety of charged (e.g. anionic) as well as non-charged monomer units and structures may be linear, branched or star-shaped. The SRP structure may also include capping groups to control molecular weight or to alter polymer properties such as surface activity. The weight average molecular weight (M_{w}) of the SRP may suitably range from about 1000 to about 20,000 and preferably ranges from about 1500 to about 10,000.

SRPs for use in the invention may suitably be selected from copolyesters of dicarboxylic acids (for example adipic acid, phthalic acid or terephthalic acid), diols (for example ethylene glycol or propylene glycol) and polydiols (for example polyethylene glycol or polypropylene glycol). The copolyester may also include monomeric units substituted with anionic groups, such as for example sulfonated isophthaloyl units. Examples of such materials include oligomeric esters produced by transesterification/oligomerization of poly(ethyleneglycol) methyl ether, dimethyl terephthalate ("DMT"), propylene glycol ("PG") and poly(ethyleneglycol) ("PEG"); partly- and fully-anionic-end-capped oligomeric esters such as oligomers from ethylene glycol ("EG"), PG, DMT and Na-3,6-dioxa-8-hydroxyoctanesulfonate; nonionic-capped block polyester oligomeric compounds such as those produced from DMT, Me-capped PEG and EG and/or PG, or a combination of DMT, EG and/or PG, Me-capped PEG and Na-dimethyl-5-sulfoisophthalate, and copolymeric blocks of ethylene terephthalate or propylene terephthalate with polyethylene oxide or polypropylene oxide terephthalate.

Other types of SRP for use in the invention include cellulosic derivatives such as hydroxyether cellulosic polymers, C₁-C₄ alkylcelluloses and C₄ hydroxyalkyl celluloses; polymers with poly(vinyl ester) hydrophobic segments such as graft copolymers of poly(vinyl ester), for example C₁-C₆ vinyl esters (such as poly(vinyl acetate)) grafted onto polyalkylene oxide backbones; poly(vinyl caprolactam) and related co-polymers with monomers such as vinyl pyrrolidone and/or dimethylaminoethyl methacrylate; and polyester-polyamide polymers prepared by condensing adipic acid, caprolactam, and polyethylene glycol.

Preferred SRPs for use in the invention include copolyesters formed by condensation of terephthalic acid ester and diol, preferably 1,2 propanediol, and further comprising an end cap formed from repeat units of alkylene oxide capped with an alkyl group. Examples of such materials have a structure corresponding to general formula (I): 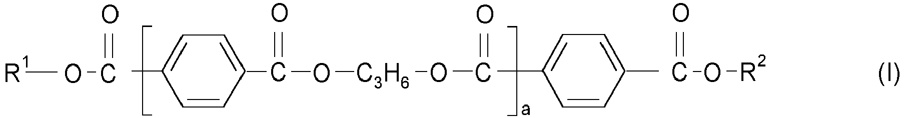
in which R¹ and R² independently of one another are X-(OC₂H₄)ₙ-(OC₃H₆)_{m ;}
in which X is C₁₋₄ alkyl and preferably methyl;
n is a number from 12 to 120, preferably from 40 to 50;
m is a number from 1 to 10, preferably from 1 to 7; and
a is a number from 4 to 9.

Because they are averages, m, n and a are not necessarily whole numbers for the polymer in bulk.

Mixtures of any of the above described materials may also be used.

The overall level of SRP, when included, may range from 0.1 to 10%, depending on the level of polymer intended for use in the final diluted composition and which is desirably from 0.3 to 7%, more preferably from 0.5 to 5% (by weight based on the total weight of the diluted composition).

Suitable soil release polymers are described in greater detail in U. S. Patent Nos. 5,574,179; 4,956,447; 4,861,512; 4,702,857, WO 2007/079850 and WO2016/005271. If employed, soil release polymers will typically be incorporated into the liquid laundry detergent compositions herein in concentrations ranging from 0.01 percent to 10 percent, more preferably from 0.1 percent to 5 percent, by weight of the composition.

### HYDROTROPES

A liquid laundry detergent composition may incorporate non-aqueous carriers such as hydrotropes, co-solvents and phase stabilizers. Such materials are typically low molecular weight, water-soluble or water-miscible organic liquids such as C1 to C5 monohydric alcohols (such as ethanol and n- or i-propanol); C2 to C6 diols (such as monopropylene glycol and dipropylene glycol); C3 to C9 triols (such as glycerol); polyethylene glycols having a weight average molecular weight (M_{w}) ranging from about 200 to 600; C1 to C3 alkanolamines such as mono-, di- and triethanolamines; and alkyl aryl sulfonates having up to 3 carbon atoms in the lower alkyl group (such as the sodium and potassium xylene, toluene, ethylbenzene and isopropyl benzene (cumene) sulfonates).

Mixtures of any of the above described materials may also be used.

Non-aqueous carriers, when included, may be present in an amount ranging from 0.1 to 20%, preferably from 2 to 15%, and more preferably from 10 to 14% (by weight based on the total weight of the composition). The level of hydrotrope used is linked to the level of surfactant and it is desirable to use hydrotrope level to manage the viscosity in such compositions. The preferred hydrotropes are monopropylene glycol and glycerol.

### COSURFACTANTS

A liquid laundry detergent composition may contain one or more cosurfactants (such as amphoteric (zwitterionic) and/or cationic surfactants) in addition to the non-soap anionic and/or nonionic detersive surfactants described above.

Specific cationic surfactants include C8 to C18 alkyl dimethyl ammonium halides and derivatives thereof in which one or two hydroxyethyl groups replace one or two of the methyl groups, and mixtures thereof. Cationic surfactant, when included, may be present in an amount ranging from 0.1 to 5% (by weight based on the total weight of the composition).

Specific amphoteric (zwitterionic) surfactants include alkyl amine oxides, alkyl betaines, alkyl amidopropyl betaines, alkyl sulfobetaines (sultaines), alkyl glycinates, alkyl carboxyglycinates, alkyl amphoacetates, alkyl amphopropionates, alkylamphoglycinates, alkyl amidopropyl hydroxysultaines, acyl taurates and acyl glutamates, having alkyl radicals containing from about 8 to about 22 carbon atoms preferably selected from C12, C14, C16, C18 and C18:1, the term "alkyl" being used to include the alkyl portion of higher acyl radicals. Amphoteric (zwitterionic) surfactant, when included, may be present in an amount ranging from 0.1 to 5% (by weight based on the total weight of the composition).

Mixtures of any of the above described materials may also be used.

### BUILDERS AND SEQUESTRANTS

The detergent compositions may also optionally contain relatively low levels of organic detergent builder or sequestrant material. Examples include the alkali metal, citrates, succinates, malonates, carboxymethyl succinates, carboxylates, polycarboxylates and polyacetyl carboxylates. Specific examples include sodium, potassium and lithium salts of oxydisuccinic acid, mellitic acid, benzene polycarboxylic acids, and citric acid. Other examples are DEQUEST^{™}, organic phosphonate type sequestering agents sold by Monsanto and alkanehydroxy phosphonates.

Other suitable organic builders include the higher molecular weight polymers and copolymers known to have builder properties. For example, such materials include appropriate polyacrylic acid, polymaleic acid, and polyacrylic/polymaleic acid copolymers and their salts, for example those sold by BASF under the name SOKALAN^{™}. If utilized, the organic builder materials may comprise from about 0.5 percent to 20 wt percent, preferably from 1 wt percent to 10 wt percent, of the composition. The preferred builder level is less than 10 wt percent and preferably less than 5 wt percent of the composition. More preferably the liquid laundry detergent formulation is a non-phosphate built laundry detergent formulation, i.e., contains less than 2 wt.%, preferably less than 1% wt. of phosphate. Most preferably the laundry detergent formulation is not built i.e. contain less than 1 wt.% of builder. A preferred sequestrant is HEDP (1 -Hydroxyethylidene -1,1,-diphosphonic acid), for example sold as Dequest 2010. Also suitable but less preferred as it gives inferior cleaning results is Dequest(R) 2066 (Diethylenetriamine penta(methylene phosphonic acid or Heptasodium DTPMP).

### POLYMERIC THICKENERS

A liquid laundry detergent composition may comprise one or more polymeric thickeners. Suitable polymeric thickeners for use in the invention include hydrophobically modified alkali swellable emulsion (HASE) copolymers. Exemplary HASE copolymers for use in the invention include linear or crosslinked copolymers that are prepared by the addition polymerization of a monomer mixture including at least one acidic vinyl monomer, such as (meth)acrylic acid (i.e. methacrylic acid and/or acrylic acid); and at least one associative monomer. The term "associative monomer" in the context of this invention denotes a monomer having an ethylenically unsaturated section (for addition polymerization with the other monomers in the mixture) and a hydrophobic section. A preferred type of associative monomer includes a polyoxyalkylene section between the ethylenically unsaturated section and the hydrophobic section. Preferred HASE copolymers for use in the invention include linear or crosslinked copolymers that are prepared by the addition polymerization of (meth)acrylic acid with (i) at least one associative monomer selected from linear or branched C₈-C₄₀ alkyl (preferably linear C₁₂-C₂₂ alkyl) polyethoxylated (meth)acrylates; and (ii) at least one further monomer selected from C₁-C₄ alkyl (meth) acrylates, polyacidic vinyl monomers (such as maleic acid, maleic anhydride and/or salts thereof) and mixtures thereof. The polyethoxylated portion of the associative monomer (i) generally comprises about 5 to about 100, preferably about 10 to about 80, and more preferably about 15 to about 60 oxyethylene repeating units.

Mixtures of any of the above described materials may also be used.

When included, a liquid laundry detergent composition will preferably comprise from 0.01 to 5% wt. of the composition but depending on the amount intended for use in the final diluted product and which is desirably from 0.1 to 3% wt. by weight based on the total weight of the diluted composition.

### FLUORESCENT AGENTS

It may be advantageous to include fluorescer in the compositions. Usually, these fluorescent agents are supplied and used in the form of their alkali metal salts, for example, the sodium salts. The total amount of the fluorescent agent or agents used in the composition is generally from 0.005 to 2 wt %, more preferably 0.01 to 0.5 wt % the composition.

Preferred classes of fluorescer are: Di-styryl biphenyl compounds, e.g. Tinopal (Trade Mark) CBS-X, Di-amine stilbene di-sulphonic acid compounds, e.g. Tinopal DMS pure Xtra, Tinopal 5BMGX, and Blankophor (Trade Mark) HRH, and Pyrazoline compounds, e.g. Blankophor SN.

Preferred fluorescers are: sodium 2 (4-styryl-3-sulfophenyl)-2H-napthol[1,2-d]triazole, disodium 4,4'-bis{[(4-anilino-6-(N methyl-N-2 hydroxyethyl) amino 1,3,5-triazin-2-yl)]amino}stilbene-2-2' disulfonate, disodium 4,4'-bis{[(4-anilino-6-morpholino-1,3,5-triazin-2-yl)]amino} stilbene-2-2' disulfonate, and disodium 4,4'-bis(2-sulfoslyryl)biphenyl.

Most preferably the fluoescer is a Di-styryl biphenyl compound, preferably Sodium 2,2'-([1,1'-biphenyl]-4,4'-diylbis(ethene-2,1-diyl))dibenzenesulfonate (CAS-No 27344-41-8).

### SHADING DYES

Shading dye can be used to improve the performance of the compositions. Preferred dyes are violet or blue. It is believed that the deposition on fabrics of a low level of a dye of these shades, masks yellowing of fabrics. A further advantage of shading dyes is that they can be used to mask any yellow tint in the composition itself.

Shading dyes are well known in the art of laundry liquid formulation.

Suitable and preferred classes of dyes include direct dyes, acid dyes, hydrophobic dyes, basic dyes, reactive dyes and dye conjugates.

Preferred examples are Disperse Violet 28, Acid Violet 50, anthraquinone dyes covalently bound to ethoxylate or propoxylated polyethylene imine as described in WO2011/047987 and WO 2012/119859 alkoxylated mono-azo thiophenes, dye with CAS-No 72749-80-5, acid blue 59, and the phenazine dye selected from: 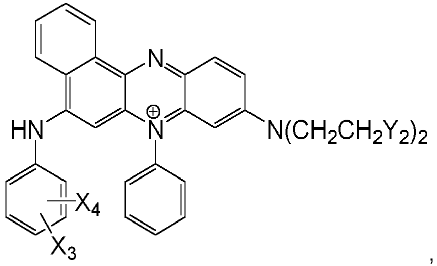 wherein:
X₃ is selected from: -H; -F; -CH₃; -C₂H₅; -OCH₃; and, -OC₂H₅;
X₄ is selected from: -H; -CH₃; -C₂H₅; -OCH₃; and, -OC₂H₅;
Y₂ is selected from: -OH; -OCH₂CH₂OH; -CH(OH)CH₂OH; -OC(O)CH₃; and, C(O)OCH_{3.}

Alkoxylated thiophene dyes are discussed in WO2013/142495 and WO2008/087497. The shading dye is preferably present is present in the composition in range from 0.0001 to 0.1wt %. Depending upon the nature of the shading dye there are preferred ranges depending upon the efficacy of the shading dye which is dependent on class and particular efficacy within any particular class.

### EXTERNAL STRUCTURANTS

Liquid laundry detergent compositions may have their rheology further modified by use of one or more external structurants which form a structuring network within the composition. Examples of such materials include hydrogenated castor oil, microfibrous cellulose and citrus pulp fibre. The presence of an external structurant may provide shear thinning rheology and may also enable materials such as encapsulates and visual cues to be suspended stably in the liquid.

### ENZYMES

A liquid laundry detergent composition may comprise an effective amount of one or more enzyme selected from the group comprising, pectate lyase, protease, amylase, cellulase, lipase, mannanase and mixtures thereof. The enzymes are preferably present with corresponding enzyme stabilizers.

### PRESERVATIVES

The composition preferably comprises a preservative.

Preferably, the composition comprises a preservative to inhibit microbial growth. For example, preservatives may optionally be included in various embodiments as a way to further boost microbial protection for gross bacteria, virus and/or fungi contamination introduced e.g., by a consumer, through a contaminated ingredient, contaminated storage container, equipment, processing step or other source. Any conventional preservative known in the art may be used. Some illustrative preservatives include: potassium sorbate, sodium benzoate, benzoic acid, phenoxyethanol, benzyl alcohol, dehydoxyacetic acid, sodium borate, boric acid, usinic acid, phenols, quaternary ammonia compounds, glycols, isothiazolinones (methyl, benzyl, chloro), DMDM hydantoin, hexidine, ethanol, IPBC, polyaminopropyl biguanide, phenylphenol, imidazolidinyl urea, parabens, formaldehyde, salicylic acid or salts, caprylyl glycol, D-glucono-1,5 lactone, sodium erythorbate, sodium hydroxymethylglycinate, peroxides, sodium sulphite, bisulphite, glucose oxidase, lacto peroxidase, and other preservatives compatible with the cleaning ingredients. Some other natural materials might also be considered like cinnamon, fruit acids, essential oils like thyme and rosemary, willow bark, aspen bark, tocopherol, curry, citrus extracts, honeysuckle, and amino acid based preservatives. Especially preferred are preservatives that do not compete with the cleaning ingredients and do not have reported health or environmental issues. Some of the more preferred preservatives are: phenoxyethanol, benzoic acid/potassium sorbate, enzymes, borates, isothiazolinones such as MIT, BIT and CIT, and the natural solutions above. In one embodiment, the preservative is present in an amount less than about 5 wt. percent based on the total weight of the cleaning composition. In another embodiment, the preservative is present in an amount from about 0.01 to about 2 wt. percent. In another embodiment, the fragrant agent is present in an amount from about 0.01 to about 1 wt. percent.

More preferably the composition comprises BIT and/or MIT at a combined level of not more than 550 ppm and more preferably at from 300 to 450 ppm. Preferably, the level of MIT does not exceed 95 ppm. Preferably, the level of BIT does not exceed 450 ppm.

Most preferably, the composition comprises benzoate salt as preservative. Preferably the benzoate salt is present at from 0.01 to 3% wt., more preferably from 0.1 to 2% wt. and most preferably from 0.5 to 1.5% wt. of the composition.

### FRAGRANCES

Fragrances are well known in the art and may be incorporated into compositions described herein.

### MICROCAPSULES

One type of microparticle suitable for use in the invention is a microcapsule. Microencapsulation may be defined as the process of surrounding or enveloping one substance within another substance on a very small scale, yielding capsules ranging from less than one micron to several hundred microns in size. The material that is encapsulated may be called the core, the active ingredient or agent, fill, payload, nucleus, or internal phase. The material encapsulating the core may be referred to as the coating, membrane, shell, or wall material.

Microcapsules typically have at least one generally spherical continuous shell surrounding the core. The shell may contain pores, vacancies or interstitial openings depending on the materials and encapsulation techniques employed. Multiple shells may be made of the same or different encapsulating materials, and may be arranged in strata of varying thicknesses around the core. Alternatively, the microcapsules may be asymmetrically and variably shaped with a quantity of smaller droplets of core material embedded throughout the microcapsule.

The shell may have a barrier function protecting the core material from the environment external to the microcapsule, but it may also act as a means of modulating the release of core materials such as fragrance. Thus, a shell may be water soluble or water swellable and fragrance release may be actuated in response to exposure of the microcapsules to a moist environment. Similarly, if a shell is temperature sensitive, a microcapsule might release fragrance in response to elevated temperatures. Microcapsules may also release fragrance in response to shear forces applied to the surface of the microcapsules.

A preferred type of polymeric microparticle suitable for use in the invention is a polymeric core-shell microcapsule in which at least one generally spherical continuous shell of polymeric material surrounds a core containing the fragrance formulation (f2). The shell will typically comprise at most 20% by weight based on the total weight of the microcapsule. The fragrance formulation (f2) will typically comprise from about 10 to about 60% and preferably from about 20 to about 40% by weight based on the total weight of the microcapsule. The amount of fragrance (f2) may be measured by taking a slurry of the microcapsules, extracting into ethanol and measuring by liquid chromatography.

Polymeric core-shell microcapsules for use in the invention may be prepared using methods known to those skilled in the art such as coacervation, interfacial polymerization, and polycondensation.

The process of coacervation typically involves encapsulation of a generally water-insoluble core material by the precipitation of colloidal material(s) onto the surface of droplets of the material. Coacervation may be simple e.g. using one colloid such as gelatin, or complex where two or possibly more colloids of opposite charge, such as gelatin and gum arabic or gelatin and carboxymethyl cellulose, are used under carefully controlled conditions of pH, temperature and concentration.

Interfacial polymerisation typically proceeds with the formation of a fine dispersion of oil droplets (the oil droplets containing the core material) in an aqueous continuous phase. The dispersed droplets form the core of the future microcapsule and the dimensions of the dispersed droplets directly determine the size of the subsequent microcapsules. Microcapsule shell-forming materials (monomers or oligomers) are contained in both the dispersed phase (oil droplets) and the aqueous continuous phase and they react together at the phase interface to build a polymeric wall around the oil droplets thereby to encapsulate the droplets and form core-shell microcapsules. An example of a core-shell microcapsule produced by this method is a polyurea microcapsule with a shell formed by reaction of diisocyanates or polyisocyanates with diamines or polyamines.

Polycondensation involves forming a dispersion or emulsion of the core material in an aqueous solution of precondensate of polymeric materials under appropriate conditions of agitation to produce capsules of a desired size, and adjusting the reaction conditions to cause condensation of the precondensate by acid catalysis, resulting in the condensate separating from solution and surrounding the dispersed core material to produce a coherent film and the desired microcapsules. An example of a core-shell microcapsule produced by this method is an aminoplast microcapsule with a shell formed from the polycondensation product of melamine (2,4,6-triamino-1,3,5-triazine) or urea with formaldehyde. Suitable cross-linking agents (e.g. toluene diisocyanate, divinyl benzene, butanediol diacrylate) may also be used and secondary wall polymers may also be used as appropriate, e.g. anhydrides and their derivatives, particularly polymers and co-polymers of maleic anhydride.

One example of a preferred polymeric core-shell microcapsule for use in the invention is an aminoplast microcapsule with an aminoplast shell surrounding a core containing the fragrance formulation (f2). More preferably such an aminoplast shell is formed from the polycondensation product of melamine with formaldehyde.

Polymeric microparticles suitable for use in the invention will generally have an average particle size between 100 nanometers and 50 microns. Particles larger than this are entering the visible range. Examples of particles in the sub-micron range include latexes and mini-emulsions with a typical size range of 100 to 600 nanometers. The preferred particle size range is in the micron range. Examples of particles in the micron range include polymeric core-shell microcapsules (such as those further described above) with a typical size range of 1 to 50 microns, preferably 5 to 30 microns. The average particle size can be determined by light scattering using a Malvern Mastersizer with the average particle size being taken as the median particle size D (0.5) value. The particle size distribution can be narrow, broad or multimodal. If necessary, the microcapsules as initially produced may be filtered or screened to produce a product of greater size uniformity.

Polymeric microparticles suitable for use in the invention may be provided with a deposition aid at the outer surface of the microparticle. Deposition aids serve to modify the properties of the exterior of the microparticle, for example to make the microparticle more substantive to a desired substrate. Desired substrates include cellulosics (including cotton) and polyesters (including those employed in the manufacture of polyester fabrics).

The deposition aid may suitably be provided at the outer surface of the microparticle by means of covalent bonding, entanglement or strong adsorption. Examples include polymeric core-shell microcapsules (such as those further described above) in which a deposition aid is attached to the outside of the shell, preferably by means of covalent bonding. While it is preferred that the deposition aid is attached directly to the outside of the shell, it may also be attached via a linking species.

Deposition aids for use in the invention may suitably be selected from polysaccharides having an affinity for cellulose. Such polysaccharides may be naturally occurring or synthetic and may have an intrinsic affinity for cellulose or may have been derivatised or otherwise modified to have an affinity for cellulose. Suitable polysaccharides have a 1-4 linked β glycan (generalised sugar) backbone structure with at least 4, and preferably at least 10 backbone residues which are β1-4 linked, such as a glucan backbone (consisting of β1-4 linked glucose residues), a mannan backbone (consisting of β1-4 linked mannose residues) or a xylan backbone (consisting of β1-4 linked xylose residues). Examples of such β1-4 linked polysaccharides include xyloglucans, glucomannans, mannans, galactomannans, β(1-3),(1-4) glucan and the xylan family incorporating glucurono-, arabino- and glucuronoarabinoxylans. Preferred β1-4 linked polysaccharides for use in the invention may be selected from xyloglucans of plant origin, such as pea xyloglucan and tamarind seed xyloglucan (TXG) (which has a β1-4 linked glucan backbone with side chains of α-D xylopyranose and β-D-galactopyranosyl-(1-2)-α-D-xylo-pyranose, both 1-6 linked to the backbone); and galactomannans of plant origin such as loc ust bean gum (LBG) (which has a mannan backbone of β1-4 linked mannose residues, with single unit galactose side chains linked α1-6 to the backbone).

Also suitable are polysaccharides which may gain an affinity for cellulose upon hydrolysis, such as cellulose mono-acetate; or modified polysaccharides with an affinity for cellulose such as hydroxypropyl cellulose, hydroxypropyl methylcellulose, hydroxyethyl methylcellulose, hydroxypropyl guar, hydroxyethyl ethylcellulose and methylcellulose.

Deposition aids for use in the invention may also be selected from phthalate containing polymers having an affinity for polyester. Such phthalate containing polymers may have one or more nonionic hydrophilic segments comprising oxyalkylene groups (such as oxyethylene, polyoxyethylene, oxypropylene or polyoxypropylene groups), and one or more hydrophobic segments comprising terephthalate groups. Typically, the oxyalkylene groups will have a degree of polymerization of from 1 to about 400, preferably from 100 to about 350, more preferably from 200 to about 300. A suitable example of a phthalate containing polymer of this type is a copolymer having random blocks of ethylene terephthalate and polyethylene oxide terephthalate.

Mixtures of any of the above described materials may also be suitable.

Deposition aids for use in the invention will generally have a weight average molecular weight (M_{w}) in the range of from about 5 kDa to about 500 kDa, preferably from about 10 kDa to about 500 kDa and more preferably from about 20 kDa to about 300 kDa.

One example of a particularly preferred polymeric core-shell microcapsule for use in the invention is an aminoplast microcapsule with a shell formed by the polycondensation of melamine with formaldehyde; surrounding a core containing the fragrance formulation (f2); in which a deposition aid is attached to the outside of the shell by means of covalent bonding. The preferred deposition aid is selected from β1-4 linked polysaccharides, and in particular the xyloglucans of plant origin, as are further described above.

The present inventors have surprisingly observed that it is possible to reduce the total level of fragrance included in the liquid laundry detergent composition without sacrificing the overall fragrance experience delivered to the consumer at key stages in the laundry process.

A reduction in the total level of fragrance is advantageous for cost and environmental reasons.

Accordingly, the total amount of fragrance formulation (f1) and fragrance formulation (f2) in the liquid laundry detergent composition suitably ranges from 0.5 to 1.4%, preferably from 0.5 to 1.2%, more preferably from 0.5 to 1% and most preferably from 0.6 to 0.9% (by weight based on the total weight of the composition).

The weight ratio of fragrance formulation (f1) to fragrance formulation (f2) in the liquid laundry detergent composition preferably ranges from 60:40 to 45:55. Particularly good results have been obtained at a weight ratio of fragrance formulation (f1) to fragrance formulation (f2) of around 50:50.

The fragrance (f1) and fragrance (f2) are typically incorporated at different stages of formation of the liquid laundry detergent composition. Typically, the discrete polymeric microparticles (e.g. microcapsules) entrapping fragrance formulation (f2) are added in the form of a slurry to a warmed base formulation comprising other components of the composition (such as surfactants and solvents). Fragrance (f1) is typically post-dosed later after the base formulation has cooled.

### FURTHER OPTIONAL INGREDIENTS

A liquid laundry detergent composition may contain further optional ingredients to enhance performance and/or consumer acceptability. Examples of such ingredients include foam boosting agents, preservatives (e.g. bactericides), polyelectrolytes, anti-shrinking agents, anti-wrinkle agents, anti-oxidants, sunscreens, anti-corrosion agents, drape imparting agents, anti-static agents, ironing aids, colorants, pearlisers and/or opacifiers, and shading dye. Each of these ingredients will be present in an amount effective to accomplish its purpose. Generally, these optional ingredients are included individually at an amount of up to 5% (by weight based on the total weight of the diluted composition) and so adjusted depending on the dilution ratio with water.

Preferably the composition comprises less than 1% alcohol and more preferably less than 0.1% alcohol.

Many of the ingredients used in embodiments of the invention may be obtained from so called black carbon sources or a more sustainable green source. The following provides a list of alternative sources for several of these ingredients and how they can be made into raw materials described herein.

### Alkyl ether sulphates

SLES and other such alkali metal alkyl ether sulphate anionic surfactants are typically obtainable by sulphating alcohol ethoxylates. These alcohol ethoxylates are typically obtainable by ethoxylating linear alcohols. Similarly, primary alkyl sulphate surfactants (PAS) can be obtained from linear alcohols directly by sulphating the linear alcohol. Accordingly, forming the linear alcohol is a central step in obtaining both PAS and alkali-metal alkyl ether sulphate surfactants.

The linear alcohols which are suitable as an intermediate step in the manufacture of alcohol ethoxylates and therefore anionic surfactants such as sodium lauryl ether sulphate can be obtained from many different sustainable sources. These include:

### Primary sugars

Primary sugars are obtained from cane sugar or sugar beet, etc., and may be fermented to form bioethanol. The bioethanol is then dehydrated to form bio-ethylene which then undergoes olefin methathesis to form alkenes. These alkenes are then processed into linear alcohols either by hydroformylation or oxidation.

An alternative process also using primary sugars to form linear alcohols can be used and where the primary sugar undergoes microbial conversion by algae to form triglycerides. These triglycerides are then hydrolysed to linear fatty acids and which are then reduced to form the linear alcohols.

### Biomass

Biomass, for example forestry products, rice husks and straw to name a few may be processed into syngas by gasification. Through a *Fischer Tropsch* reaction these are processed into alkanes, which in turn are dehydrogenated to form olefins. These olefins may be processed in the same manner as the alkenes described above [primary sugars].

An alternative process turns the same biomass into polysaccharides by steam explosion which may be enzymatically degraded into secondary sugars. These secondary sugars are then fermented to form bioethanol which in turn is dehydrated to form bio-ethylene. This bio-ethylene is then processed into linear alcohols as described above [primary sugars].

### Waste Plastics

Waste plastic is pyrolyzed to form pyrolysed oils. This is then fractioned to form linear alkanes which are dehydrogenated to form alkenes. These alkenes are processed as described above [primary sugars].

Alternatively, the pyrolyzed oils are cracked to form ethylene which is then processed to form the required alkenes by olefin metathesis. These are then processed into linear alcohols as described above [primary sugars].

### Municipal Solid Waste

MSW is turned into syngas by gasification. From syngas it may be processed as described above [primary sugars] or it may be turned into ethanol by enzymatic processes before being dehydrogenated into ethylene. The ethylene may then be turned into linear alcohols by the *Ziegler Process.*

The MSW may also be turned into pyrolysis oil by gasification and then fractioned to form alkanes. These alkanes are then dehydrogenated to form olefins and then linear alcohols.

### Marine Carbon

There are various carbon sources from marine flora such as seaweed and kelp. From such marine flora the triglycerides can be separated from the source and which is then hydrolysed to form the fatty acids which are reduced to linear alcohols in the usual manner.

Alternatively, the raw material can be separated into polysaccharides which are enzymatically degraded to form secondary sugars. These may be fermented to form bioethanol and then processed as described above [Primary Sugars].

### Waste Oils

Waste oils such as used cooking oil can be physically separated into the triglycerides which are split to form linear fatty acids and then linear alcohols as described above. Alternatively, the used cooking oil may be subjected to the Neste Process whereby the oil is catalytically cracked to form bio-ethylene. This is then processed as described above.

### Methane Capture

Methane capture methods capture methane from landfill sites or from fossil fuel production. The methane may be formed into syngas by gasification. The syngas may be processed as described above whereby the syngas is turned into methanol (*Fischer Tropsch* reaction) and then olefins before being turned into linear alcohols by hydroformylation oxidation.

Alternatively, the syngas may be turned into alkanes and then olefins by *Fischer Tropsch* and then dehydrogenation.

### Carbon Capture

Carbon dioxide may be captured by any of a variety of processes which are all well known. The carbon dioxide may be turned into carbon monoxide by a reverse water gas shift reaction and which in turn may be turned into syngas using hydrogen gas in an electrolytic reaction. The syngas is then processed as described above and is either turned into methanol and/or alkanes before being reacted to form olefins.

Alternatively, the captured carbon dioxide is mixed with hydrogen gas before being enzymatically processed to form ethanol. This is a process which has been developed by Lanzatech. From here the ethanol is turned into ethylene and then processed into olefins and then linear alcohols as described above.

The above processes may also be used to obtain the C16/18 chains of the C16/18 alcohol ethoxylate and/or the C16/18 ether sulfates.

### LAS

One of the other main surfactants commonly used in cleaning compositions, in particular laundry compositions is LAS (linear alkyl benzene sulphonate).

The key intermediate compound in the manufacture of LAS is the relevant alkene. These alkenes (olefins) may be produced by any of the methods described above and may be formed from primary sugars, biomass, waste plastic, MSW, carbon capture, methane capture, marine carbon to name a few.

Whereas in the processes described above the olefin is processed to form linear alcohols by hydroformylation and oxidation instead, the olefin is reacted with benzene and then sulphonate to form the LAS.

The invention will be further described with the following non-limiting examples.

### Example

Crude palm oil and low erucic acid rapeseed (canola) oil were obtained from agricultural sources. The oils were mixed in various ratios and transesterified to the corresponding methyl ester, and the methyl esters separated from the glycerol and residual methanol. The methyl esters were reduced to the corresponding alcohol and the distribution of carbon chain length measured by GC. The values are given below.

| | **Comparative** US 5,672,781 **Example 2 & 3** | | **According to the Invention** | | | |
|---|---|---|---|---|---|---|
| **Chain length (wt.%)** | PKO | 7:3 | 2:8 | 3:7 | 4:6 | 5:5 |
| | | PKO/RSO | CPO:RSO | CPO:RSO | CPO:RSO | CPO:RSO |
| **C12** | 0 | 0 | 0.1 | 0.1 | 0.2 | 0.2 |
| **C14** | 0.1 | 0 | 0.3 | 0.4 | 0.5 | 0.6 |
| **C16** | 12.3 | 23.9 | 12.8 | 16.7 | 20.6 | 24.5 |
| **C18** | 86.2 | 74.7 | 83.1 | 78.9 | 74.7 | 70.6 |
| **Distillation required?** | yes | yes | no | no | no | no |

PKO is palm kernel oil;
RSO is low erucic acid rapeseed oil;
CPO crude palm oil

The iodine value for all samples were measured and if required further hydrogenation applied till a value of 70-75 obtained. Under these conditions the predominate C18 fraction is oleyl.

The inventive mixtures of RSO and CPO provide matching carbon chain distribution without the need for distillation and the removal of waste fractions.

Fatty acid methyl esters (FAME) of rapeseed oil were prepared by transesterification reaction catalysed by potassium hydroxide (KOH). The reaction took place in a 2-I three-necked round-bottomed flask, equipped with a stirrer and a condenser. The amount of catalyst used was 1 wt.% compared to the starting rapeseed oil with 9:1 methanol to oil molar ratio. KOH and methanol were added to rapeseed oil and stirred at 60 °C for 2 h and cooled down. After that, the lower glycerine phase was removed. The upper phase was washed with 60 °C distilled water several times to remove any remaining KOH, methanol, and possible soap. Finally, it was dried using a rotary evaporator at 60 °C, to remove the remaining washing water. The FAME composition was determined by GC.

## Claims

1. A process to produce an oleyl alcohol comprising the following steps:-
a) hydrogenation of a fatty acid, a fatty acid methyl ester or a combination thereof to provide a fatty alcohol;
wherein the fatty acid, the fatty acid methyl ester oil or combination thereof contain oleyl chains obtained from a mixture of 1:9 to 6:4, preferably 2:8 to 5:5 oil (1) and oil (2), where oil (1) is selected from palm oil, cottonseed oil or mixtures thereof, oil (2) is selected from tall oil, olive oil, low erucic rapeseed oil, sunflower seed oil variants containing greater than 60 wt.% oleic acid or mixtures thereof; and wherein the process does not include distillation.

2. A process according to claim 1, wherein oil (1) is palm oil, and oil (2) is selected from low erucic rapeseed oil and sunflower seed oil variants containing greater than 60 wt.% oleic acid or mixtures thereof, preferably wherein oil (2) is low erucic rapeseed oil.

3. A process according to claim 1, wherein oil (1) is palm oil, and oil (2) is low erucic rapeseed oil.

4. A process according to any one of claims 1 to 3, wherein the oleyl alcohol is converted to a surfactant, preferably to a surfactant selected from: oleyl sulfate, oleyl alcohol ethoxylate, oleyl ether sulfate, oleyl ether carboxylate, and oleyl sulfosuccinate, preferably oleyl alcohol ethoxylate, oleyl ether sulfate and oleyl ether carboxylate.

5. A process according to any one of claims 1 to 4, wherein the fatty acid methyl ester is produced by transesterification of triglyceride to fatty acid methyl esters followed by removal of glycerol.

## Patentansprüche

1. Verfahren zur Herstellung von Oleylalkohol, umfassend die folgenden Schritte:
a) Hydrierung einer Fettsäure, eines Fettsäuremethylesters oder einer Kombination davon, um einen Fettalkohol bereitzustellen;
wobei die Fettsäure, das Fettsäuremethylesteröl oder die Kombination davon Oleylketten enthält, erhalten aus einer Mischung von Öl (1) und Öl (2) im Verhältnis 1:9 bis 6:4, bevorzugt 2:8 bis 5:5, wobei das Öl (1) unter Palmöl, Baumwollsamenöl oder Mischungen davon ausgewählt ist, das Öl (2) unter Tallöl, Olivenöl, erucasäurearmem Rapsöl, Sonnenblumenkernölvarianten, enthaltend mehr als 60 Gew.-% Ölsäure, oder Mischungen davon ausgewählt ist und wobei das Verfahren keine Destillation umfasst.

2. Verfahren nach Anspruch 1, wobei das Öl (1) Palmöl ist und das Öl (2) unter erucasäurearmem Rapsöl und Sonnenblumenkernölvarianten, die mehr als 60 Gew.-% Ölsäure enthalten, oder Mischungen davon ausgewählt ist, wobei das Öl (2) vorzugsweise ein erucasäurearmes Rapsöl ist.

3. Verfahren nach Anspruch 1, wobei das Öl (1) Palmöl ist und das Öl (2) erucasäurearmes Rapsöl ist.

4. Verfahren nach einem der Ansprüche 1 bis 3, wobei der Oleylalkohol in ein Tensid überführt wird, bevorzugt in ein Tensid, ausgewählt unter Oleylsulfat, Oleylalkoholethoxylat, Oleylethersulfat, Oleylethercarboxylat und Oleylsulfosuccinat, bevorzugt aus Oleylalkoholethoxylat, Oleylethersulfat und Oleylethercarboxylat.

5. Verfahren nach einem der Ansprüche 1 bis 4, wobei der Fettsäuremethylester durch Umesterung von Triglycerid zu Fettsäuremethylestern und anschließendes Entfernen von Glycerin hergestellt wird.

## Revendications

1. Procédé pour produire un alcool oléylique comprenant les étapes suivantes :
a) hydrogénation d'un acide gras, d'un ester méthylique d'acide gras ou d'une combinaison de ceux-ci pour fournir un alcool gras ;
dans lequel l'acide gras, l'huile d'ester méthylique d'acide gras ou la combinaison de ceux-ci contiennent des chaînes oléyles obtenues à partir d'un mélange de 1:9 à 6:4, de préférence de 2:8 à 5:5 d'huile (1) et d'huile (2), où l'huile (1) est choisie parmi l'huile de palme, l'huile de graines de coton ou des mélanges de celles-ci, l'huile (2) est choisie parmi l'huile de pin, l'huile d'olive, l'huile de colza à faible teneur en acide érucique, les variantes d'huile de graines de tournesol contenant plus de 60 % en poids d'acide oléique ou des mélanges de celles-ci ; et dans lequel le procédé n'inclut pas de distillation.

2. Procédé selon la revendication 1, dans lequel l'huile (1) est de l'huile de palme, et l'huile (2) est choisie parmi l'huile de colza à faible teneur en acide érucique et les variantes d'huile de graines de tournesol contenant plus de 60 % en poids d'acide oléique ou des mélanges de celles-ci, de préférence dans lequel l'huile (2) est de l'huile de colza à faible teneur en acide érucique.

3. Procédé selon la revendication 1, dans lequel l'huile (1) est de l'huile de palme, et l'huile (2) est de l'huile de colza à faible teneur en acide érucique.

4. Procédé selon l'une quelconque des revendications 1 à 3, dans lequel l'alcool oléylique est converti en un tensioactif, de préférence en un tensioactif choisi parmi : le sulfate d'oléyle, l'éthoxylate d'alcool oléylique, le sulfate d'éther oléylique, le carboxylate d'éther oléylique, et le sulfosuccinate d'oléyle, de préférence l'éthoxylate d'alcool oléylique, le sulfate d'éther oléylique et le carboxylate d'éther oléylique.

5. Procédé selon l'une quelconque des revendications 1 à 4, dans lequel l'ester méthylique d'acide gras est produit par transestérification de triglycéride en esters méthyliques d'acide gras suivie d'une élimination de glycérol.
